(19)

Europäisches Patentamt

European Patent Office

Office européen des brevets

(11)     EP 2 979 625 B1

## (12)     EUROPEAN PATENT SPECIFICATION

(45) Date of publication and mention
     of the grant of the patent:
     **13.06.2018   Bulletin 2018/24**

(51) Int Cl.:
     *A61B 5/00* *(2006.01)*          *B62J 99/00* *(2009.01)*
     *A63B 22/00* *(2006.01)*         *B62K 19/36* *(2006.01)*
     *B62K 21/00* *(2006.01)*         *B62K 21/18* *(2006.01)*
     *G05G 23/00* *(2006.01)*

(21) Application number: **14382295.5**

(22) Date of filing: **30.07.2014**

(54) **System for determining a suitable position of a cyclist on a bicycle and method implemented with said system**

System zur Bestimmung einer geeigneten Position eines Radfahrers auf einem Fahrrad und mit besagtem System implementiertes Verfahren

Système pour déterminer une position appropriée d'un cycliste sur une bicyclette et procédé mis en oeuvre avec ce système

(84) Designated Contracting States:
     **AL AT BE BG CH CY CZ DE DK EE ES FI FR GB
     GR HR HU IE IS IT LI LT LU LV MC MK MT NL NO
     PL PT RO RS SE SI SK SM TR**

(43) Date of publication of application:
     **03.02.2016   Bulletin 2016/05**

(73) Proprietor: **F4 Biomechanics, S.L.**
     **48140 Igorre (ES)**

(72) Inventors:
     • **Ortuondo Gorostiza-Goiza, Jon Ander**
       **48140 Igorre (ES)**

     • **De Arriba Martin, Pedro**
       **35010 Las Palmas de Gran Canaria (ES)**

(74) Representative: **Igartua, Ismael**
     **Galbaian S.Coop.**
     **Polo de Innovación Garaia**
     **Goiru Kalea 1 - P.O. Box 213**
     **20500 Arrasate-Mondragón (ES)**

(56) References cited:
     **EP-A1- 2 353 983          WO-A1-2014/066994
     FR-A1- 2 495 307          FR-A1- 2 671 324
     US-A1- 2008 246 250       US-A1- 2012 202 653**

**Description**

TECHNICAL FIELD

The present invention relates to systems for determining a suitable position of a cyclist on a bicycle, and methods for determining the position of a cyclist on a bicycle.

PRIOR ART

US 2012/0202653 A discloses a system for determining a suitable position of a cyclist according to the preamble of claim 1.

The suitable position of a cyclist on a bicycle is the first step to be taken when practicing the sport of cycling to the extent that said position should even determine whether or not to buy the bike, or at the very least the adaptation of the bicycle. The position of the cyclist on the bicycle involves balancing different sections which, as a whole, will have a direct effect on the cyclist's physical performance, comfort on the bicycle and ergonomics on said bicycle in order to avoid injuries. The position which allows bringing together the best performance, and/or comfort and/or ergonomic conditions according to the cyclist's requirements, is referred to as the suitable position.

In the cyclist's position on the bicycle, said cyclist has three contact points, i.e.: the saddle, the hoods in the case of road bikes, or the grips in the case of mountain bikes, or armrest in the case of aero bikes, hereinafter referred to as hoods in any of the three cases, and the pedals. Determining the position of said contact points in the suitable position of the cyclist on the bicycle allows defining measurements in the bicycle referred to as biometric measurements.

WO2014/066994 A1 describes a system for determining the suitable position of a cyclist on a bicycle, the bicycle being of the type comprising a frame, a fork coupled to the frame, a handlebar assembly comprising a stem and a handlebar coupled to the stem, the stem being coupled to the fork, a pair of hoods coupled to the handlebar, a saddle, and a seatpost coupled at one end to the saddle and at the other end to the frame. The system comprises a handlebar assembly stem adjusting device, a saddle adjusting device, and control and information exchange means connected to the handlebar assembly stem adjusting device, and/or to the saddle adjusting device.

DISCLOSURE OF THE INVENTION

The object of the invention is to provide a system for determining a suitable position of a cyclist on a bicycle and a method for determining a suitable position of a cyclist on a bicycle, as defined in the claims.

One aspect of the invention relates to a system for determining a suitable position of a cyclist on a bicycle, usually the cyclist's own bicycle or a similar bicycle to define the fitting. The bicycle comprises a frame, a fork coupled to the frame, a handlebar assembly comprising a stem and a handlebar coupled to the stem, the stem being coupled to the fork, a pair of hoods coupled to the handlebar, a saddle, and a seatpost coupled at one end to the saddle and at the other end to the frame, the system comprising a handlebar assembly adjusting device, a saddle adjusting device, and control and information exchange means connected to the handlebar assembly adjusting device, and/or to the saddle adjusting device. The handlebar assembly adjusting device replaces the handlebar assembly of the bicycle, and the hoods are coupled to the handlebar assembly adjusting device, said handlebar assembly adjusting device providing an interface between the fork and the hoods, and the handlebar assembly adjusting device allowing movement of the position of the hoods in a longitudinal direction, in a vertical direction, in a transverse direction, and at a tilt angle, to the suitable position of the cyclist on the bicycle.

Another aspect of the invention relates to a method for determining a suitable position of a cyclist on a bicycle implemented with a system such as the one defined above.

In the system of the state of the art, the handlebar assembly stem adjusting device only replaces the stem of the bicycle, the handlebar being coupled to said device, and the hoods are not coupled to the handlebar. Said handlebar assembly stem adjusting device therefore provides an interface between the fork and the handlebar. The position of the handlebar is thus adjusted to the suitable position, but not the position of the hoods, which is where the cyclist supports himself/herself most of the time. The handlebar assembly stem adjusting device also allows performing only longitudinal and vertical movements of the handlebar, so the individual defining the suitable position of the cyclist on the bicycle, usually a biomechanist, will be limited when defining said position.

In the system and in the method implemented with said system of the invention, the handlebar assembly adjusting device replaces the handlebar assembly of the bicycle, i.e., it replaces the assembly formed by the stem and the handlebar of the bicycle, and the hoods are coupled to the handlebar assembly adjusting device. The cyclist therefore goes through the session for defining the fitting in real conditions, being supported on the hoods of the bicycle. The handlebar assembly adjusting device therefore provides an interface between the fork and the hoods of the bicycle. The individual in charge of the fitting adjusts the position of the hoods to a suitable position of the cyclist on the bicycle. To that end, the handlebar assembly adjusting device allows moving the hoods in a longitudinal direction, in a vertical direction and in a transverse direction, and at a tilt angle, so the individual in charge of the fitting is not limited and can rapidly define the suitable position of the hoods of the bicycle with precise results, which allows providing feedback to the cyclist with quality data, and allows performing the fitting with complete mobility.

DESCRIPTION OF THE DRAWINGS

Figure 1 shows a side view of a road bike with the contact points of the cyclist on said bike.

Figure 2 shows a side view of a mountain bike with the contact points of the cyclist on said bike.

Figure 3 shows a side view of an aero-type bike with the contact points of the cyclist on said bike.

Figure 4 shows a side view of an embodiment of the system of the invention, mounted in the bicycle of Figure 1.

Figures 5a, 5b, 5c show first and second perspective views and a side view, respectively, of the handlebar assembly adjusting device of Figure 4.

Figures 6a, 6b, and 6c show a first perspective view, a second perspective view, and a side view, respectively, of the saddle adjusting device of Figure 4.

Figures 7-12 show initial measurements of components of the bicycle of Figure 1.

Figures 13-14 show initial - final configuration measurements of the bicycle of Figure 1 which can be measured by the cyclist.

Figure 15 shows measurements of the seatpost and hoods of the bicycle of Figure 1 for defining the initial position of the saddle and of the hoods in the movement step for moving the saddle and hoods.

Figures 16-17 show manipulation measurements of the bicycle of Figure 1, which are manipulated in the movement step for moving the saddle and hoods.

Figures 18-19 show biometric measurements of the bicycle of Figure 1, of the contact points of the saddle, of the hoods, and of the pedals of the cyclist on the bicycle when said cyclist is in the suitable position.

Figures 20-21 show suggested measurements of components of the bicycle of Figure 1.

DETAILED DISCLOSURE OF THE INVENTION

The suitable fitting of the cyclist on the bicycle is one of the first steps to be taken when practicing the sport of cycling to the extent that said fitting should even determine whether or not to buy the bike, or at the very least the adaptation of the bicycle to the characteristics of the cyclist by defining the so-called biometric measurements. The position of the cyclist on the bicycle involves balancing different sections which, as a whole, have a direct effect on the cyclist's physical performance, comfort and ergonomics.

To this day, the adjustment of the suitable position of the cyclist on the bicycle is done manually by means of trial and error, i.e., by changing the different measurements in the bicycle until adjusting it to the cyclist. Fitting is also performed with the aid of stationary systems and/or devices, such as stationary bicycles, in which the cyclist is pedaling while the most important measurements of the bicycle, such as the position of the saddle and the handlebar, are automatically adjusted. The fitting of the cyclist on a normal bicycle, on a bicycle belonging to the cyclist or on a bicycle that has been lent by someone else is also adjusted, defining the position of the contact points on the saddle and on the handlebar.

However, these different systems or devices take up a lot of time and a lot of mistakes are also made, making such systems or devices imprecise. Therefore, the results must be translated to the cyclist's bicycle by means of complex calculations, and the measurements obtained in the cyclist's bicycle must later be adjusted, or else the contact points of the cyclist on the bicycle will not be accurately and precisely defined, as in the case of the support on the saddle, if the reference is not the suitable reference, or the support of the hands on the handlebar, and not on the hoods of the bicycle, which is where cyclists actually support themselves. This occurs when adjusting the measurements the first time and on successive occasions.

With the methodology normally used for performing the fitting of the cyclist, measurements of the bicycle which correspond, though not always precisely enough, to the biometric measurements defining the suitable position of the cyclist on said bicycle are obtained. However, when said measurements of the bicycle cannot be reproduced in the bicycle, components of the bicycle must be changed so that they allow reproducing the biometric measurements in the bicycle. Methods that allow knowing which components are required for the change and what measurements are used are known, but they are obtained in an approximate manner, for example based on bicycle frames approximating the optimal frames which the cyclist would require, but are not based on the frame or on the bicycle belonging to said cyclist.

The system for determining the position of a cyclist on a bicycle of the invention and the method implemented with said system solve the aforementioned problems.

There are many different types of bicycles on the market. Out of said types of bicycle, the most commonly used for performing a sporting activity on moving bicycles for cyclists in general, and for experienced cyclists and professional cyclists in particular, are referred to as road bikes, mountain bikes, and aero bikes for time trials or for triathlons. Any of said bicycles is identified with reference number 100.

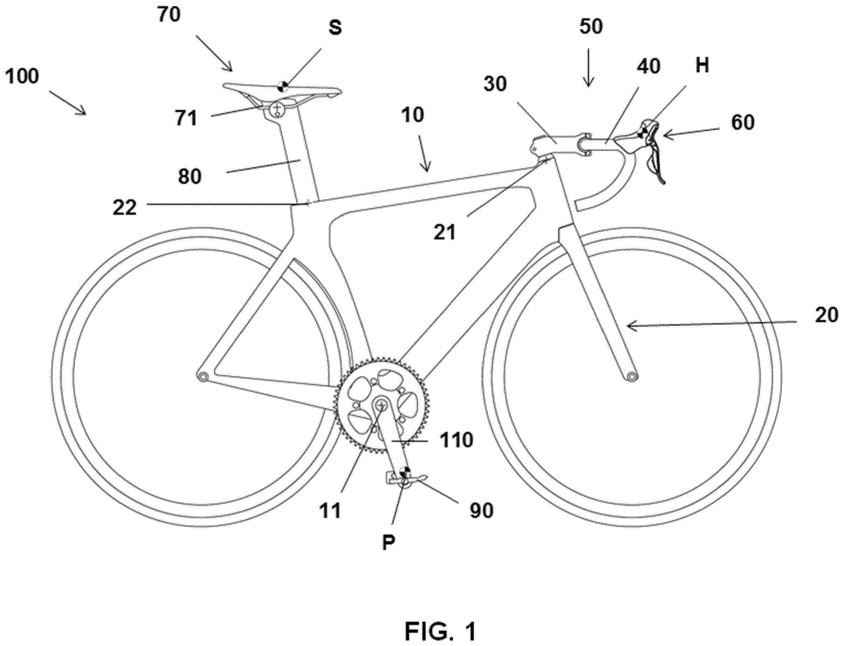

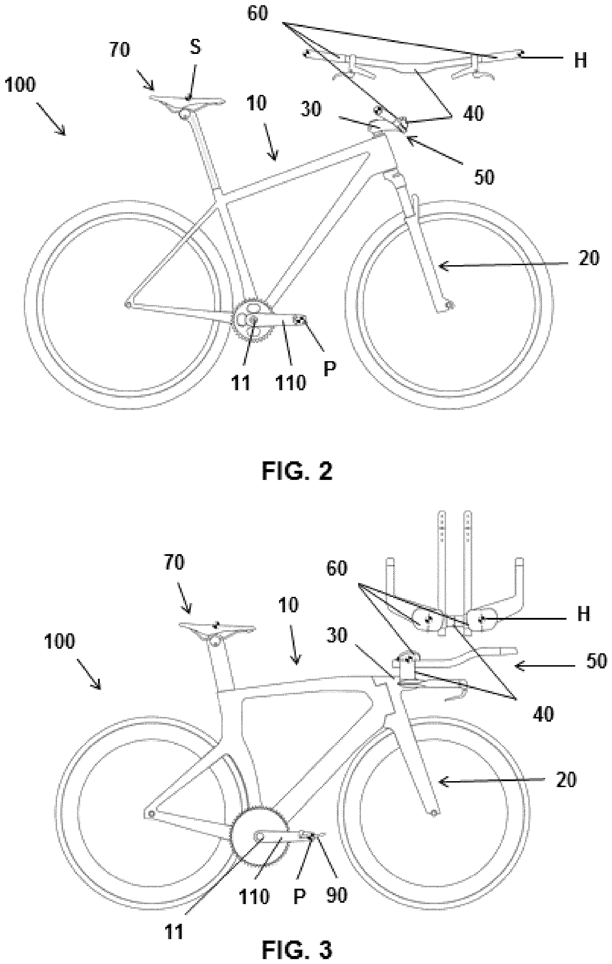

Figure 1 shows a side view of a road bike 100, Figure 2 shows a side view of a mountain bike 100, and Figure 3 shows an aero bike 100. Said bicycles comprise a frame 10 which is the central structure of the bicycle 100, a fork 20 coupled to the frame 10 and from which there projects an end 21, a handlebar assembly 50 comprising a stem 30 and a handlebar 40 coupled to the stem 30, the stem 30 being coupled to the end 21 of the fork 20, a pair of hoods 60 being coupled to the handlebar 40, where the cyclist's hands are supported throughout most of the pedaling time, a saddle 70 where the cyclist's backside is supported, a seatpost 80 coupled at one end to the saddle 70 and at the other end to the frame 10, and the height of said seatpost 80 can be adjusted, a pair of cranks 110, and at one end of said cranks 110 a pair of pedals 90, the other end of each crank 110 in a crank shaft 11 being coupled to the frame 10.

When the bicycle is a mountain bike 100, the grips, generically referred to as hoods 60, are attached to the handlebar 40 in a prolongation of said handlebar 40, the cyclist's hands being supported throughout most of the pedaling time on said hoods 60. When the bicycle is an aero-type bike 100, the armrest, generically referred to as hoods 60, are used for supporting the cyclist's forearms throughout most of the pedaling time, the lower part of said hoods 60 being attached to the handlebar 40.

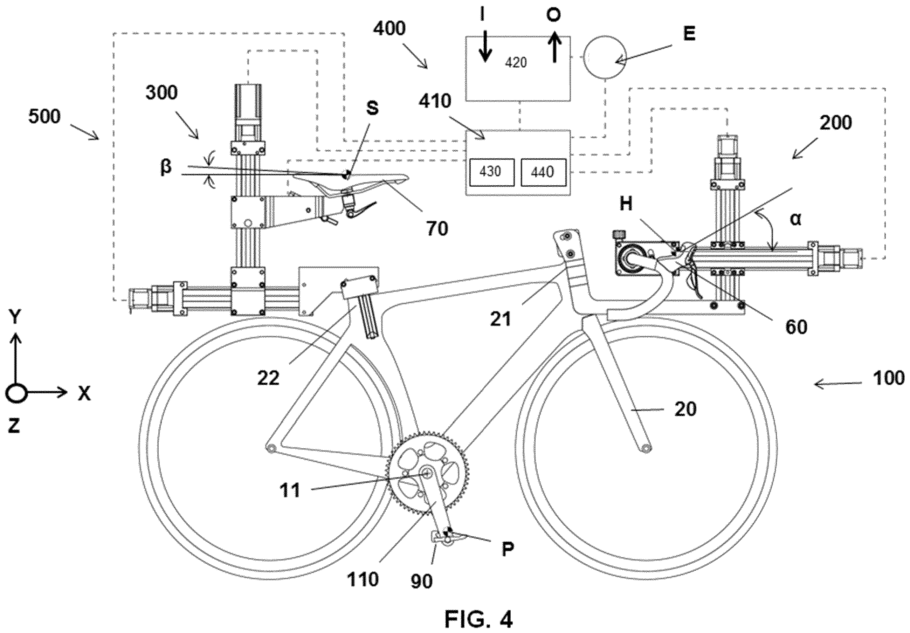

Figure 4 shows a side view of an embodiment of the system 500 of the invention mounted on the bicycle 100 of Figure 1. Said system 500 comprises a handlebar assembly adjusting device 200, a saddle adjusting device 300, and control and information exchange means 400 connected to the handlebar assembly adjusting device 200 and to the saddle adjusting device 300. The connection is a wired connection with electrical connection lines and data lines, but it can also be wireless (not shown in the drawings), control in this case being by remote control.

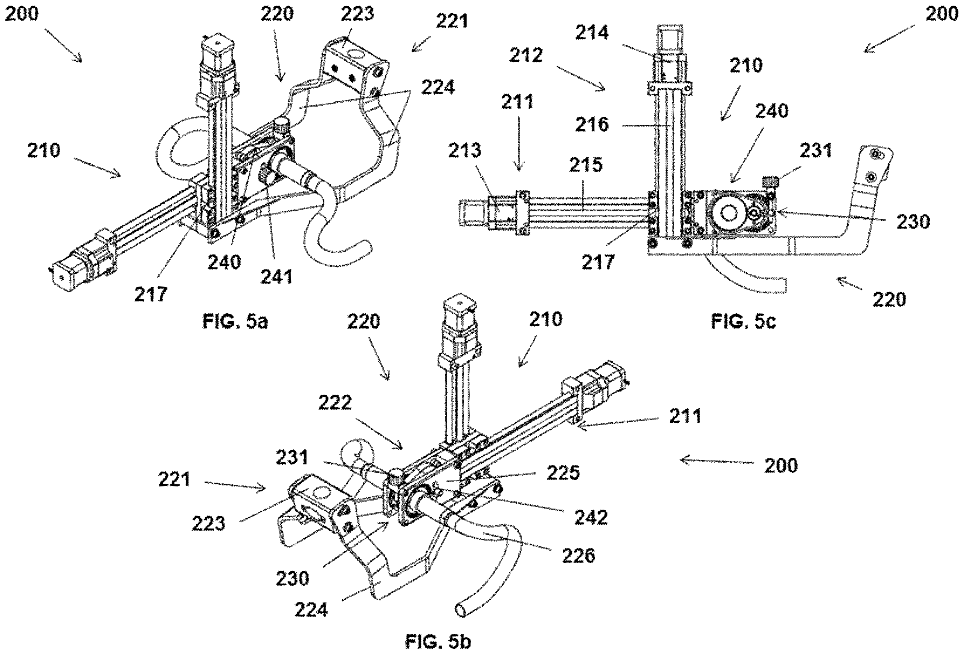

Figures 5a, 5b, and 5c show a first perspective view, a second perspective view and a side view, respectively, of the handlebar assembly adjusting device 200 of Figure 4. The handlebar assembly adjusting device 200 replaces the handlebar assembly 50 of the bicycle 100 shown in Figure 1. For said replacement, the stem 30 is removed from its coupling with the fork 20, which in turn has the handlebar 40 coupled thereto, and the handlebar assembly adjusting device 200 is coupled at the end 21 of the fork 20. Then either the hoods 60 of the bicycle 100, or hoods similar to hoods 60, are coupled to the handlebar assembly adjusting device 200. An interface is therefore obtained between the fork 20 and the hoods 60 of the bicycle 100 with said handlebar assembly adjusting device 200.

The handlebar assembly adjusting device 200 comprises a handlebar assembly operating unit 210, and a handlebar assembly adjustable unit 220 which is coupled to the handlebar assembly operating unit 210 and is also coupled to the fork 20. The handlebar assembly operating unit 210 controls the movements of the handlebar assembly adjustable unit 220, allowing said handlebar assembly adjustable unit 220 to make a movement in a vertical direction Y, a movement in a longitudinal direction X, a movement in a transverse direction Z, and a variation in a tilt angle $\alpha$.

The handlebar assembly adjustable unit 220 comprises a handlebar unit 222 and a support 221. The handlebar unit 222 comprises a handlebar support 225, which in this embodiment is a case which allows supporting different elements, coupled to the handlebar assembly operating unit 210, as will be explained below. The handlebar unit 222 also comprises a handlebar member 226, which is a handlebar that replaces the function of the handlebar 40 of the bicycle 100, coupled to the handlebar support 225. The characteristics of the handlebar member 226 and the coupling thereof to the handlebar support 225 allow being able to open or close said handlebar member 226 in the transverse direction Z of the bicycle 100, and being able to tilt at a variable angle $\alpha$. The hoods 60 of the bicycle 100 are coupled to the handlebar member 226 in the same position they had in the bicycle 100 coupled to the handlebar 40.

The handlebar assembly adjusting device 200 thereby allows movement of the position of the hoods 60 in the longitudinal direction X, in the vertical direction Y, and in the transverse direction Z, and furthermore at a tilt angle $\alpha$.

The handlebar assembly operating unit 210 comprises a depth actuator 230 for the handlebar member 226, which allows providing a variation in the width of said handlebar member 226 in the transverse direction Z. Said handlebar member 226 can thus be freely increased or decreased in width, the hoods 60 moving in the same way together with the handlebar member 226. The depth actuator 230 is coupled to the handlebar member 226 by means that are known in the state of the art, such as by means of threaded spindles, for example, such that actuation in one direction allows movement of the handlebar member 226 in the transverse direction Z. In the shown embodiment of the handlebar assembly adjusting device 200, the depth actuator 230 is arranged in the handlebar support 225 and comprises a manual actuation member 231 arranged in said handlebar support 225, allowing direct access for the user of the system 500. In other embodiments (not shown in the drawings), the depth actuator 230 can comprise motor-driven operating means, such that the variation in the width of the handlebar member 226, and therefore of the hoods 60, can even be done remotely.

In this embodiment, the handlebar assembly operating unit 210 also comprises a tilt actuator 240 for the handlebar member 226, which allows providing a variation in the tilt angle α of said handlebar member 226 with respect to the horizontal. Said handlebar member 226 can therefore be freely tilted, the tilt of the hoods 60 varying in the same way together with the handlebar member 226. The tilt actuator 240 is coupled to the handlebar member 226 by means that are known in the state of the art, such as by means of an assembly formed by levers and cams, for example, such that actuation in one direction allows the rotation of the handlebar member 226 in the direction of the tilt angle α. In the shown embodiment of the handlebar assembly adjusting device 200, the tilt actuator 240 is arranged in the handlebar support 225 and comprises a manual actuation member 241 arranged in said handlebar support 225, allowing direct access for the user of the system 500. The tilt actuator 240 also comprises an angle indicator 242, indicating the angle rotated by the handlebar member 226, arranged in the handlebar support 225. The user of the system 500 therefore knows which variation in the tilt angle α is being applied. In other embodiments (not shown in the drawings), the tilt actuator 240 can comprise motor-driven operating means, such that the variation in the tilt angle α of the handlebar member 226, and therefore of the hoods 60, can even be done remotely.

In this embodiment, the handlebar assembly operating unit 210 also comprises a horizontal actuator 211 to control the handlebar assembly adjustable unit 220, which allows providing movement in the longitudinal direction X of the handlebar assembly adjustable unit 220, and therefore of the hoods 60, with respect to the fork 20. It also comprises a vertical actuator 212 to control the handlebar assembly adjustable unit 220, which allows providing movement in the vertical direction Y of the handlebar assembly adjustable unit 220, and therefore of the hoods 60, with respect to the fork 20.

In this embodiment, the horizontal actuator 211 and the vertical actuator 212 of the handlebar assembly operating unit 210 are similar actuators, each comprising a drive motor 213 and 214, a support structure 215 and 216, and a linear movement member (not shown in the drawings), respectively. The drive motors 213 and 214 are arranged at one end of the respective structures 215 and 216, these structures being fixed support shafts with closure elements surrounding them. Linear movement members, i.e., threaded spindles in this embodiment, are arranged inside the structures 215 and 216. The vertical actuator 212 further comprises a support member 217 surrounding the support structure 216 coupled to said vertical actuator 212, which allows the sliding thereof along said structure 216 of the vertical actuator 212. This support member 217 is attached on one hand to the linear movement member of the vertical actuator 212, and on the other hand, it is coupled to the support structure 215 of the horizontal actuator 211, supporting the horizontal actuator 211. Therefore, the support member 217 allows coupling between the horizontal actuator 211 and the vertical actuator 212 of the handlebar assembly operating unit 210, the horizontal actuator 211, and therefore the handlebar unit 222, being able to move in the vertical direction Y.

The support 221 of the handlebar assembly adjustable unit 220 comprises a fork coupling member 223, which in this embodiment is a housing with a hole, which allows coupling the support 221 to the end 21 of the fork 20 of the bicycle 100. The support 221 also comprises an arm 224 that is L-shaped when seen from the side, with two side arms which are attached at one end to the fork coupling member 223, a segment of which descends in the vertical direction Y and another segment of which moves forward in the longitudinal direction X, and they are attached to one another at the other end, supporting at this other end the vertical actuator 212 at an end opposite to where the drive motor 213 is located. The handlebar support 225 of the handlebar unit 222 is coupled to the horizontal actuator 211 at an end opposite to where the drive motor 214 is located, such that when the horizontal actuator 211 moves, the handlebar assembly adjustable unit 220 moves in the horizontal direction X.

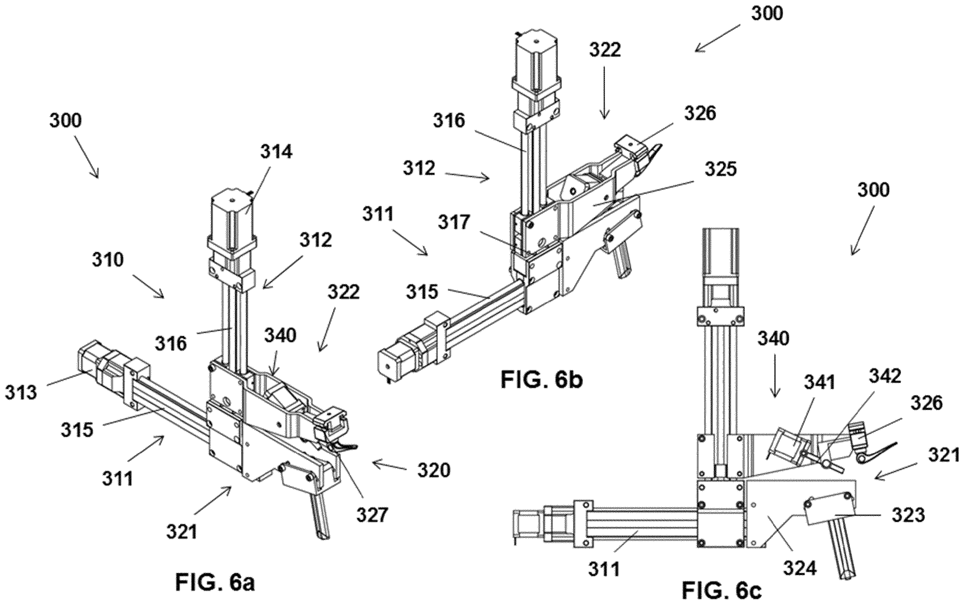

Figures 6a, 6b and 6c show a first perspective view, a second perspective view and a side view of the saddle adjusting device 300 of Figure 4, respectively. The saddle adjusting device 300 replaces the seatpost 80 of the bicycle 100 shown in Figure 1. For said replacement, in this embodiment of the invention the saddle 70 is removed from its coupling with the seatpost 80; the seatpost 80 is removed completely, introducing it in the frame 10, and the saddle adjusting device 300 is coupled at one end 22 of the frame 10, which is a fixed reference. The saddle 70 of the bicycle 100 is then coupled to the saddle adjusting device 300. An interface between the frame 10 and the saddle 70 of the bicycle 100 is therefore obtained with said saddle adjusting device 300.

The saddle adjusting device 300 comprises a seatpost operating unit 310 and a seatpost adjustable unit 320 which is coupled to the seatpost operating unit 310, to the frame 10 at end 22, and also to the saddle 70. The seatpost operating unit 310 controls the movements of the seatpost adjustable unit 320, allowing said seatpost adjustable unit 320 to make a movement in the vertical direction Y, a movement in the longitudinal direction X, and a variation in the a tilt angle β, and therefore the saddle 70 makes the same movements and the same variation in the tilt angle.

The seatpost operating unit 310 comprises a horizontal actuator 311 to control the seatpost adjustable unit 320, which allows providing movement of the seatpost adjustable unit 320, and therefore of the saddle 70, in the longitudinal direction X with respect to the frame 10. It also comprises a vertical actuator 312 to control the seatpost adjustable unit 320, which allows providing movement of the seatpost adjustable unit 320, and therefore of the saddle 70, in the vertical direction Y with respect to the frame 10.

In this embodiment, the horizontal actuator 311 and the vertical actuator 312 of the seatpost operating unit 310 are similar actuators, each comprising a drive motor 313 and 314, a support structure 315 and 316, and a linear movement member (not shown in the drawings), respectively. The drive motors 313 and 314 are arranged at one end of the respective structures 315 and 316, these structures being fixed support shafts with closure elements surrounding them. Linear movement members, i.e., threaded spindles in this embodiment, are arranged inside the structures 315 and 316. The horizontal actuator 311 further comprises a support member 317 surrounding the support structure 315 coupled to said horizontal actuator 311, which allows the sliding thereof along said structure 315 of the horizontal actuator 311. This support member 317 is attached on one hand to the linear movement member of the horizontal actuator 311, and on the other hand, it is coupled to the support structure 316 of the vertical actuator 312, supporting the vertical actuator 312. Therefore, the support member 317 allows coupling between the horizontal actuator 311 and the vertical actuator 312 of the seatpost operating unit 310, the vertical actuator 312, and therefore the seatpost adjustable unit 320, being able to move in the horizontal direction X.

The seatpost adjustable unit 320 comprises a support 321 and a saddle unit 322. The saddle unit 322 comprises a saddle support 325, which in this embodiment is an assembly formed by two strips mounted parallel to one another, with an inner space that allows supporting different elements, and it is coupled to the saddle 70 as will be explained below. Said saddle support 325 on the other hand is coupled at one end to the vertical actuator 312, being attached to the linear movement member of said vertical actuator 312. The saddle support 325 comprises a saddle coupling 326 attached to the saddle support 325 at the other end thereof, the saddle 70 of the bicycle 100 being coupled to the saddle coupling 326. The saddle 70 comprises rails 71 in the lower part thereof, which allow coupling with the seatpost 80. When the saddle adjusting device 300 replaces the seatpost 80, the saddle 70 is coupled to the saddle support 325 in the saddle coupling 326. To that end, said saddle coupling 326 is designed such that it has grooves 327 in which the rails 71 of the saddle 70 are coupled. Therefore, the saddle support 325, and therefore the saddle 70, can move in the vertical direction Y. Likewise, since the horizontal actuator 311 and the vertical actuator 312 are coupled to one another, when the horizontal actuator moves the support member 317, the saddle 70 also moves in the horizontal direction X.

The support 321 of the seatpost adjustable unit 320, which in this embodiment is an assembly formed by two parallel strips comprising an inner space therebetween, said support 321 comprising a frame coupling member 323 at one end. This frame coupling member 323 comprises coupling means, for example rods with screws, which allows coupling the support 321 to the end 22 of the frame 10 of the bicycle 100. The support 321 also comprises an arm 324, as a continuation of the frame coupling member 323, attached at the other end to the horizontal actuator 311, supporting it at the end of the horizontal actuator 311 opposite to where the drive motor 313 is located.

The seatpost operating unit 310 also comprises a tilt actuator 340 for the saddle 70 to provide a variation in the tilt angle β of said saddle 70 with respect to the horizontal. The tilt actuator 340 is arranged inside the saddle unit 322 and comprises a drive motor 341 and an angular movement member 342 in this embodiment. This angular movement member 342 is a spindle with a spindle nut at one end, the other end of the spindle being coupled to the drive motor 341. When the saddle is attached to the saddle unit 322, coupled in the saddle coupling 326 of the saddle support 325, the spindle nut of the angular movement member 342 is supported in the saddle 70 in the lower part thereof, such that the when the angular movement member 342 is operated by the drive motor 341, it makes the spindle nut rotate, producing the variation in the tilt angle β of the saddle 70. In this embodiment, the tilt actuator 340 of the saddle 70 is electrically connected and can be actuated directly or by remote control, but it can also be a manual tilt actuator (not shown in the drawings), in which case it comprises a manual actuation member arranged in the saddle unit 322. In any of the embodiments, the tilt actuator can comprise an indicator showing the angle rotated by the saddle 70, which would be arranged in the saddle unit 322.

Like the tilt actuator 240 for the handlebar assembly operating unit 210, this tilt actuator 340 can be non-motor driven (embodiment not shown in the drawings), comprising in said embodiment a manual actuation member arranged in the saddle support 325.

The control and information exchange means 400 shown in Figure 4 comprise a control unit 410 to control the handlebar assembly adjusting device 200 and the saddle adjusting device 300 in their movements in the longitudinal direction X, the vertical direction Y, the transverse direction Z, and at the tilt angle α and β of the hoods 60 and the saddle 70, respectively. The control unit 410 also allows picking up the coordinates obtained, in the movements and at the tilt angles mentioned above, with respect to the crank shaft 11 of the bicycle 100.

The control and information exchange means 400 also comprise an interface 420 connected to the control unit 410 to make selections relating to the system 500 allowing the user of the system 500 to define the movements to be made with the saddle 70 and the hoods 60. The interface 420 also allows inputting I initial measurements and characteristics of components MCI of the bicycle 100, initial measurements and characteristics of the bicycle 100 itself, and initial measurements and characteristics of bicycle components and initial measurements and characteristics of bicycles existing on the market. It also allows outputting O the biometric measurements of the contact points S, M, P of the cyclist on the saddle 70, on the hoods 60, and on the pedals 90 of the bicycle 100 when the cyclist is in the suitable position on said bicycle 100. It also allows outputting O final configuration measurements of the bicycle which are those that can be measured by the cyclist, and final measurements of components MCF of the bicycle 100, allowing the user or cyclist to modify the coordinates of the contact points S, M, P in the longitudinal direction X, in the vertical direction Y, in the transverse direction Z, and at the tilt angle $\alpha$ and $\beta$, of the hoods 60 and of the saddle 70, respectively.

The control and information exchange means 400 also comprise data storage means 430 for storing the input information I and output information O, and processing means 440 for treating the input information I and determining the output information O.

The control and information exchange means 400 are connected to either grid-connected or independent power supply means E, or another type of power allowing the control and information exchange means 400 to work.

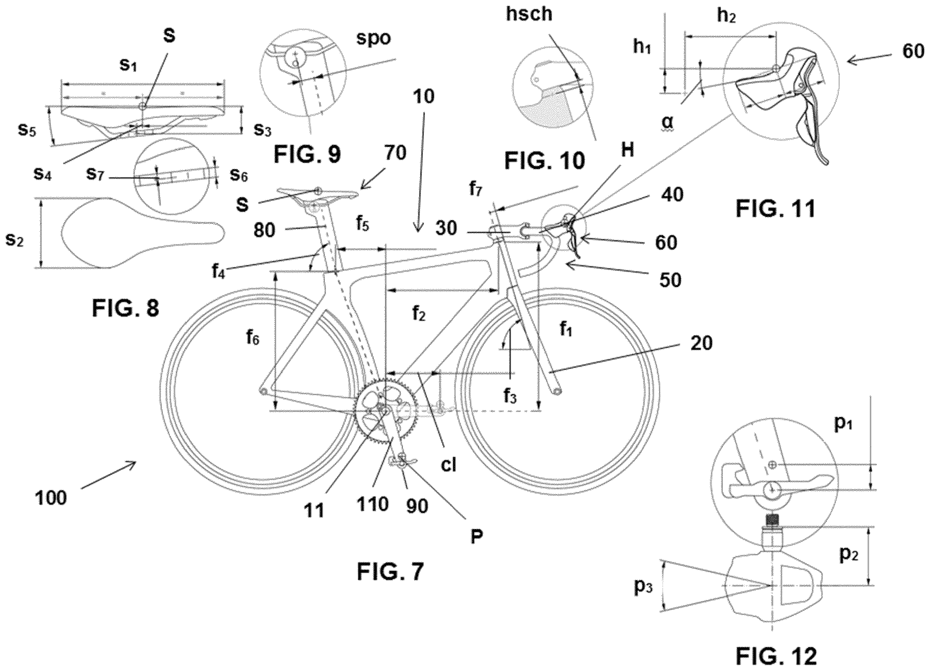

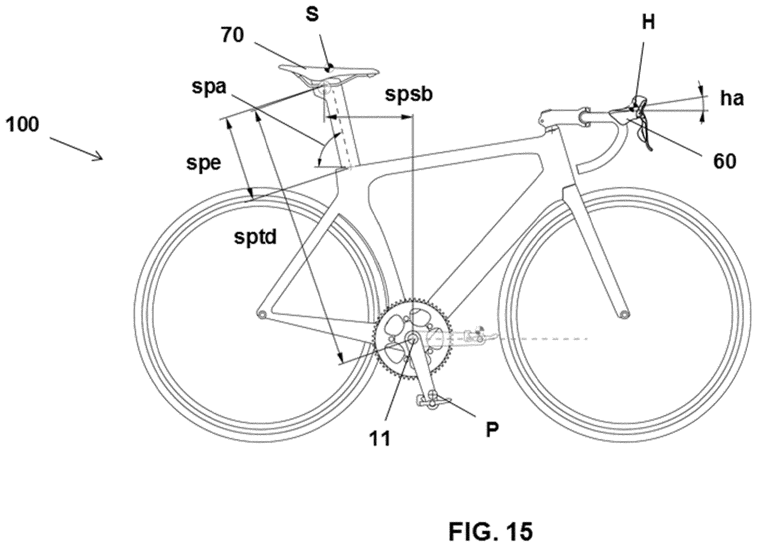

The method for determining a suitable position of a cyclist on a bicycle 100 of the invention is implemented, for example, with a system 500 such as the one shown in Figures 1-6 and described above. Figures 7-21 show the bicycle 100 and different components of said bicycle 100, defining measurements aiding in the interpretation of the steps of an embodiment of the method of the invention for the fitting of a cyclist on a bicycle 100. Said method comprises:

- a step for entering data about the cyclist S0 when the cyclist is new in the definition of fitting;

- an input step I for inputting information S1 about initial measurements and characteristics of components (MCI) of the bicycle 100 and about bicycle components on the market with the aid of the interface 420 and the data storage means 430.

The components the MCI of which are inputted include: frame 10, hoods 60, saddle 70, seatpost 80, pedals 90, and cranks 110. The entered information includes the measurements of said components and measurements of the bicycle 100 with respect to the crank shaft 11. The characteristics of the components are the make, model, year of manufacture and size. These measurements are shown in Figures 7-12 and are for the bicycle 100: height of the end of the frame $f_1$, reach of the end of the frame $f_2$, angle of the end of the frame $f_3$, angle of the seatpost with respect to the horizontal $f_4$, insertion of the saddle in the frame in the longitudinal direction X $f_5$, insertion of the saddle in the frame in the vertical direction Y $f_6$, and radius of the end of the frame $f_7$. For the saddle 70: length $s_1$, width $s_2$, rail height $s_3$, longitudinal movement of the center of the rail with respect to the center of the saddle $s_4$, rail angle $s_5$, rail thickness $s_6$, and mean rail thickness $s_7$. For the seatpost 80: movement of securing the rail of the saddle with the seatpost with respect to the longitudinal axis of the seatpost sp. For the end 21 of the fork 20: height of the cone of the end of the fork hsch. For the hoods 60: hood support point drop with respect to the handlebar axis $h_1$, hood support point reach with respect to the handlebar axis $h_2$, hood support point tilt angle with respect to the horizontal $\alpha$. For the pedals 90: height $p_1$, width $p_2$, and floating bracket angle $p_3$. For the crank 110: length cl;

- a movement step S2 for moving the saddle 70 and hoods 60 of the bicycle 100. The original handlebar assembly 50 and saddle 70 are removed from the bicycle 100, and the handlebar assembly adjusting device 200 and the saddle adjusting device 300 are coupled to the fork 20 and frame 10, respectively, coupling the hoods 60 and saddle 70 to the handlebar assembly adjusting device 200 and to the saddle adjusting device 300, respectively, arranging said devices in an initial position or zero position to learn in which position the support point H on the hoods 60 and the support point S on the saddle 70 are located with the aid of processing means 440. Then, with the cyclist mounted on the bicycle 100, the position of the saddle 70 is moved in the longitudinal direction X, in the vertical direction Y, and at the tilt angle $\beta$, and the position of the hoods 60 is moved in the longitudinal direction X, in the vertical direction Y, in the transverse direction Z, and at the tilt angle $\alpha$, to the suitable position of the cyclist, and the coordinates in X, Y, and Z, and the tilt angle $\alpha$ and $\beta$ of the movement of the saddle 70 and of the hoods 60 from the initial start position in the session for defining the fitting up to obtaining the suitable position of the cyclist are picked up with the aid of the control means 410. The coordinates in the longitudinal direction X and in the vertical direction Y of the pedals 90 are also picked up.

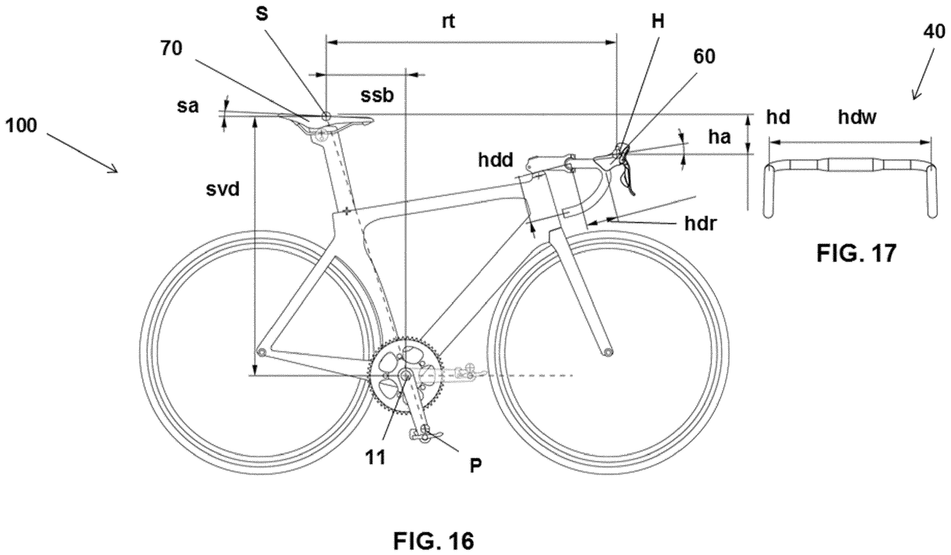

The dimensions that are manipulated to obtain the movement of the saddle 70 and hoods 60 are shown in Figures 16-17, and they include: saddle angle sa, saddle vertical distance svd, saddle setback ssb, total reach rt, hood angle ha, handlebar reach hdr, handlebar drop hdd, total drop between saddle support point and hood support point dt, and handlebar width hdw.

- a step for determining biometric measurements S3 of the bicycle 100 at the contact points S, M, P of the cyclist on the bicycle 100 with respect to the crank shaft 11.

After the manipulation performed in the movement step S2 of the saddle 70 and hoods 60 to the suitable position of the cyclist on the bicycle 100, the biometric measurements of the bicycle 100 are determined based on the MCI of the bicycle 100 and of the bicycle 100 itself which are entered in the control and information exchange means 400, on known measurements of the handlebar assembly adjusting device 200 and on known measurements of the saddle adjusting device 300, and on the coordinates in the longitudinal direction X, in the vertical direction Y, and at the tilt angle β of the movement of the saddle 70, and on the coordinates in the longitudinal direction X, in the vertical direction Y, in the transverse direction Z, and at the tilt angle α of the movement of the hoods 60, from the initial position until the suitable position of the cyclist, with the aid of the processing means 440.

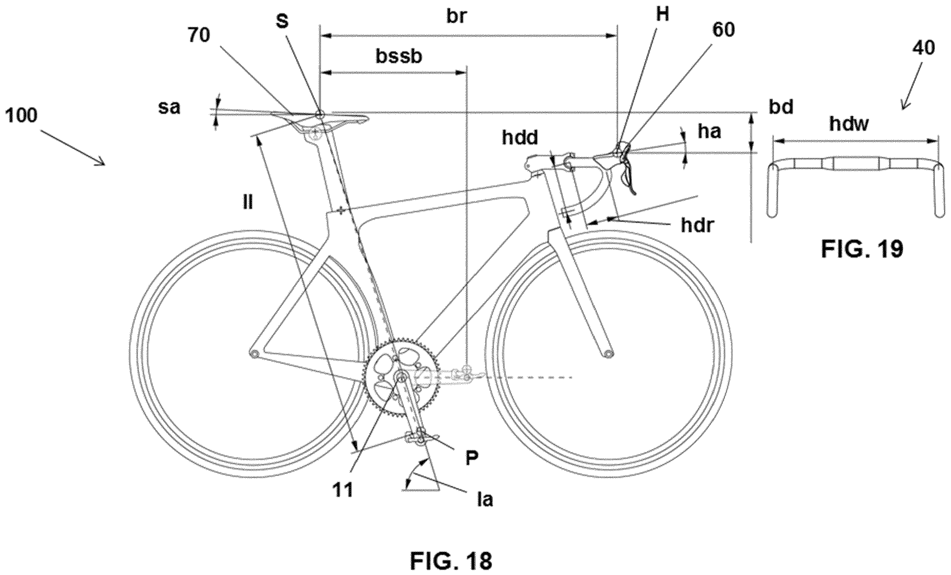

The biometric measurements are shown in Figures 18-19, and they include: saddle angle sa, leg length ll, biometric saddle setback bssb, biometric reach br, hood angle ha, handlebar reach hdr, handlebar drop hdd, biometric drop between saddle support point and hood support point bd, handlebar width hdw, and leg angle la, and

- a step for defining final configuration measurements of the bicycle MBF and suggested measurements of components MCF of the bicycle S4, which can be measured by the cyclist based on the biometric measurements and on the MCI of the bicycle 100, with the aid of the processing means 440.

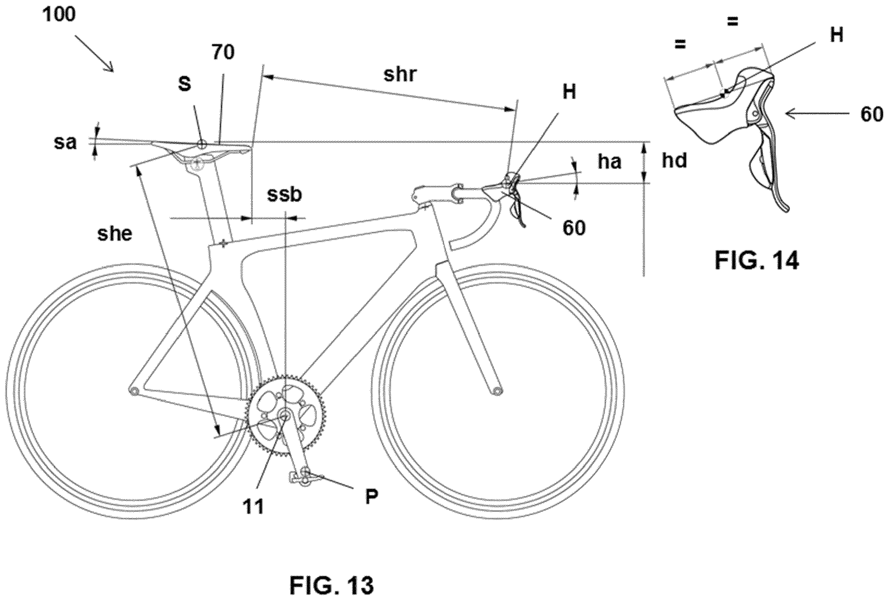

The final configuration measurements of the bicycle MBF are shown in Figures 13-14, and they include: saddle angle sa, height of the saddle she, saddle setback ssb, saddle-hoods reach shr, hood angle ha, hood support point drop with respect to the saddle support point hd, and distance between ends of hoods with respect to the support point =.

With these measurements MBF, the cyclist or the biomechanist can configure the bicycle 100 to the biometric measurements at any time. The following is true:

"Optimal fitting (biometric)" = "Measurements of components of the bicycle" + (together with) "Final configuration measurements MBF",

such that knowing that the "Optimal fitting (biometric)" is maintained for one and the same cyclist, if the bicycle 100 or any of its components changes, the new "Final configuration measurements MBF" are obtained from the following equation:

"Final configuration measurements MBF" = "Optimal fitting (biometric)" – (deducting) "Measurements of components of the bicycle".

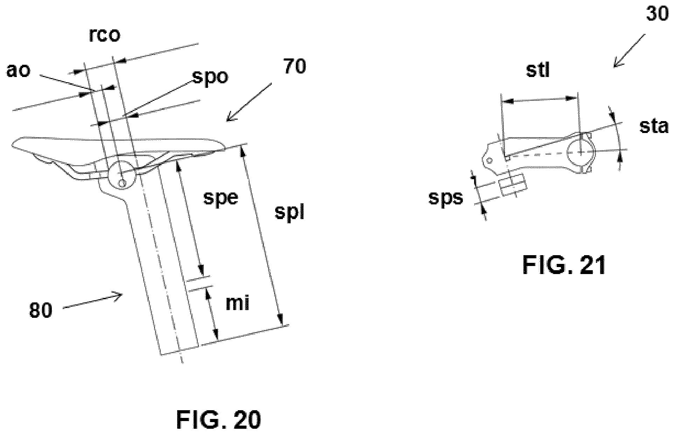

The components the suggested measurements MCF of which are defined in this step S4 are: the stem 30, the handlebar 40, and the seatpost 80, and they are shown in Figures 20-21 and in Figures 18-19. Said measurements MCF are for the seatpost 80: movement for securing the rail of the saddle with the seatpost, with respect to the longitudinal axis of the seatpost spo, movement of the seatpost assembly ao, movement of the center of the rail of the saddle with respect to the longitudinal axis of the seatpost rco, seatpost length spl, seatpost extension spe, and minimum insertion of the seatpost mi. For the stem 30: stem length stl, stem angle sta, and spacer stack sps. For the handlebar 40: width hdw, reach hdr, and drop hdd.

The following conditions are met:

$$spl > spe + mi,$$

$$rco = spo + ao.$$

If the suggested measurements of the components MCF of the bicycle 100 correspond with the MCI of the bicycle 100, it means that the bicycle 100 with its original components can be configured with the biometric measurements determined in step S3. If the suggested measurements of the components MCF of the bicycle 100 do not correspond with the MCI of the bicycle 100, it means that the bicycle 100 with its original components cannot be configured with the biometric measurements, and a warning appears on the interface 420 together with the components and the MCF needed to configure the bicycle 100 with the biometric measurements.

The input step I for inputting information S1 comprises:

- a phase for entering initial configuration measurements of the bicycle MBI P11, equivalent to the final configuration measurements of the bicycle MBF defined in step S4 and shown in Figures 13-14, said initial configuration measurements of the bicycle MBI corresponding to measurements of the bicycle 100 that can be measured when it is in the initial situation, without installing the system 500, said measurements MBI being entered with the aid of the interface 420. Therefore, when the session for defining the fitting begins, it starts from the initial position or zero position, the initial configuration measurements of the bicycle MBI, stored in the data storage means 430, allowing movement of the position of the saddle 70 and of the hoods 60 from the initial position to a starting position corresponding to the initial configuration measurements of the bicycle MBI. Step S2 for movement of the position of the saddle 70 and of the hoods 60 to the suitable position of the cyclist begins from this starting position.

Movement step S2 comprises:

- a phase for defining the initial position of the saddle 70 and hoods 60 P21 after installing the system 500, coupling the saddle 70 to the saddle adjusting device 300, and coupling the hoods 60 to the handlebar assembly adjusting device 200, the coordinates in the longitudinal direction X, in the vertical direction Y, and the tilt angle $\beta$ of the support point S of the saddle 70 being calculated, and the coordinates in the longitudinal direction X, in the vertical direction Y, in the transverse direction Z, and the tilt angle $\alpha$ of the support point H of the hoods 60 of the initial position with respect to the crank shaft 11 being calculated based on the initial measurements and characteristics of components MCI of the bicycle 100, on the known measurements of the handlebar assembly adjusting device 200 and on the known measurements of the saddle adjusting device 300 when they are coupled to the fork 20 and to the frame 10, respectively, and on the hood tilt angle ha, on the coordinates in the longitudinal direction X and the vertical direction Y of the end 22 of the frame 10, with the aid of processing means 440.

**Claims**

1. System for determining a suitable position of a cyclist on a bicycle, the bicycle (100) being of the type comprising a frame (10), a fork (20) coupled to the frame (10), a handlebar assembly (50) comprising a stem (30) and a handlebar (40) coupled to the stem (30), the stem (30) being coupled to the fork (20), a pair of hoods (60) removably coupled to the handlebar (40), a saddle (70), and a seatpost (80) coupled at one end to the saddle (70), and at the other end to the frame (10), the system (500) comprising a handlebar assembly adjusting device (200), a saddle adjusting device (300), and control and information exchange means (400) connected to the handlebar assembly adjusting device (200) and/or to the saddle adjusting device (300), **characterized in that** the handlebar assembly adjusting device (200) is configured to replace the handlebar assembly (50) of the bicycle (100), said handlebar assembly adjusting device (200) being configured such that the pair of hoods (60) of the bicycle (100) can be coupled thereto, said handlebar assembly adjusting device (200) being configured to be coupled at an end of the fork so as to provide an interface between the fork (20) and the hoods (60), and the handlebar assembly adjusting device (200) allowing movement of the position of the hoods (60) in a longitudinal direction (X), in a vertical direction (Y) and in a transverse direction (Z), and at a tilt angle ($\alpha$), to the suitable position of the cyclist on the bicycle (100).

2. System according to claim 1, wherein the handlebar assembly adjusting device (200) comprises a handlebar assembly operating unit (210), and a handlebar assembly adjustable unit (220), coupled to the handlebar assembly operating unit (210) and coupled to the fork (20), wherein the handlebar assembly operating unit (210) controls the handlebar assembly adjustable unit (220) to provide movement in the vertical direction (Y) and/or movement in the longitudinal direction (X), and wherein the handlebar assembly adjustable unit (220) comprises a handlebar unit (222), the handlebar unit (222) comprising a handlebar support (225) coupled to the handlebar assembly operating unit (210), and a handlebar member (226) coupled to the handlebar support (225), the hoods (60) of the bicycle (100) being coupled to the handlebar member (226).

3. System according to claim 2, wherein the handlebar assembly operating unit (210) comprises a depth actuator (230) for the handlebar member (226) to provide a variation in the width of said handlebar member (226) in the transverse direction (Z), the depth actuator (230) being coupled to the handlebar member (226), and said depth actuator (230) comprising a manual actuation member (231) arranged in the handlebar support (225).

4. System according to claim 2 or 3, wherein the handlebar assembly operating unit (210) comprises a tilt actuator (240) for the handlebar member (226), to provide a variation in the tilt angle ($\alpha$) of said handlebar member (226) with respect to the horizontal, the tilt actuator (240) being coupled to the handlebar member (226), and said tilt actuator (240) comprising a manual actuation member (241) arranged in the handlebar support (225), and an indicator (242) showing the angle rotated by the handlebar member (226), arranged in the handlebar support (225).

5. System according to any of claims 2 to 4, wherein the handlebar assembly operating unit (210) comprises a horizontal actuator (211) to control the handlebar assembly adjustable unit (220), to provide movement in the longitudinal direction (X) of the hoods (60) with respect to the fork (20), and a vertical actuator (212) to control the handlebar assembly adjustable unit (220), to provide movement in the vertical direction (Y) of the hoods (60) with respect to the fork (20).

6. System according to claim 5, wherein the horizontal actuator (211) and the vertical actuator (212) of the handlebar assembly operating unit (210) each comprises at least one drive motor (213, 214), a support structure (215, 216), and a linear movement member, respectively, the vertical actuator (212) further comprising a support member (217), supporting the support structure (215) of the horizontal actuator (211), coupled to the support structure (216) of the vertical actuator (212), said support member (217) allowing coupling between the horizontal actuator (211) and the vertical actuator (212) of the handlebar assembly operating unit (210).

7. System according to claim 5 or 6, wherein the handlebar assembly adjustable unit (220) comprises a support (221), said support (221) comprising a fork coupling member (223) which allows coupling the support (221) to an end (21) of the fork (20) of the bicycle (100), and at least one arm (224) attached at one end to the fork coupling member (223), and supporting at the other end the vertical actuator (212) of the handlebar assembly operating unit (210), the handlebar support (225) of the handlebar unit (222) being coupled to the horizontal actuator (211) of the handlebar assembly operating unit (210).

8. System according to any of the preceding claims, wherein the saddle adjusting device (300) is configured to replace the seatpost (80) of the bicycle (100), said saddle adjusting device (300) providing an interface between the frame (10) and the saddle (70), and the saddle adjusting device (300) allowing movement of the position of the saddle (70) in the longitudinal direction (X) and in the vertical direction (Y), and at a tilt angle ($\beta$), to a suitable position of the cyclist on the bicycle (100), the saddle adjusting device (300) comprising a seatpost operating unit (310), and a seatpost adjustable unit (320), coupled to the seatpost operating unit (310) and coupled to the frame (10), the saddle (70) being coupled to the seatpost adjustable unit (320), wherein the seatpost operating unit (310) controls the seatpost adjustable unit (320) to provide movement in the vertical direction (Y) and/or movement in the longitudinal direction (X).

9. System according to claim 8, wherein the seatpost operating unit (310) comprises a tilt actuator (340) for the saddle (70), to provide a variation in the tilt angle ($\beta$) of said saddle (70) with respect to the horizontal, the tilt actuator (340) comprising a drive motor (341) and an angular movement member (342) coupled at one end to the drive motor (341) and cooperating at the other end with the saddle (70).

10. System according to claim 8 or 9, wherein the seatpost operating unit (310) comprises a horizontal actuator (311) to control the seatpost adjustable unit (320) to provide movement in the longitudinal direction (X) of the saddle (70) with respect to the frame (10), and a vertical actuator (312) to control the seatpost adjustable unit (320) to provide movement in the vertical direction (Y) of the saddle (70) with respect to the frame (10), the horizontal actuator (311) and the vertical actuator (312) each comprising at least one drive motor (313, 314), a support structure (315, 316), and a linear movement member, respectively, the horizontal actuator (311) further comprising a support member (317) supporting the support structure (316) of the vertical actuator (312), coupled to the support structure (315) of the horizontal actuator (311), said support member (317) allowing coupling between the horizontal actuator (311) and the vertical actuator (312) of the seatpost operating unit (310).

11. System according to claim 10, wherein the seatpost adjustable unit (320) comprises a support (321) and a saddle unit (322), the support (321) comprising a frame coupling member (323) which allows coupling the support (321) to one end (22) of the frame (10) of the bicycle (100), and at least one arm (324) attached at one end to the frame coupling member (323), and supports at the other end the horizontal actuator (311) for the seatpost operating unit (310), the saddle unit (322) comprising a saddle support (325) coupled to the vertical actuator (312) for the seatpost operating unit (310), and the saddle support (325) comprising a saddle coupling (326) attached to the saddle support (325), the saddle (70) of the bicycle (100) being coupled to the saddle coupling (326).

12. System according to any of claims 8 to 11, wherein the control and information exchange means (400) comprise a control unit (410) to control the handlebar assembly adjusting device (200) and the saddle adjusting device (300), to pick up the coordinates in the longitudinal direction (X), in the vertical direction (Y), and in the transverse direction (Z), and the tilt angle ($\alpha$) of the hoods (60), and/or the coordinates in the longitudinal direction (X) and in the vertical direction (Y), and the tilt angle ($\beta$) of the saddle (70), with respect to a crank shaft (11) of the bicycle (100), an interface (420) connected to the control unit (410), to make selections relating to the system (500), to input (I) initial measurements and characteristics of components (MCI) of the bicycle (100), initial measurements and characteristics of the bicycle (100) itself, initial measurements and characteristics of bicycle components and initial measurements and characteristics of bicycles existing on the market, and to output (O) biometric measurements of the contact points (S, M, P) of the cyclist on the saddle (70), on the hoods (60), and on pedals (90) of the bicycle (100), when the cyclist is in the suitable position, final configuration measurements of the bicycle, and final measurements of components (MCF) of the bicycle (100), allowing the user to modify the coordinates in the longitudinal direction (X), in the vertical direction (Y), and in the transverse direction (Z), as well as the tilt angle ($\alpha$) of the hoods (60), and/or to modify the coordinates in the longitudinal direction (X) and in the vertical direction (Y), and the tilt angle ($\beta$) of the saddle (70), data storage means (430) for storing the input information (I) and output information (O), and processing means (440) for treating the input information (I) and determining the output information (O).

13. Method for determining a suitable position of a cyclist on a bicycle, implemented with a system (500) according to any of the preceding claims, **characterized in that** it comprises:

- an input step (I) for inputting information (S1) about initial measurements and characteristics of components (MCI) of the bicycle (100) and about bicycle components on the market, the components being the frame (10), the hoods (60), the saddle (70), the seatpost (80), pedals (90), and cranks (110), and the information entered are the measurements of said components and the characteristics of said components, such as the make, model, year of manufacture and size;
- a movement step (S2) for moving the saddle (70) and/or hoods (60) with the system (500) mounted on the bicycle (100), and with the cyclist mounted on the bicycle (100), coupling the hoods (60) and the saddle (70) to the handlebar assembly adjusting device (200), and to the saddle adjusting device (300), respectively, moving the position of the saddle (70) in the longitudinal and vertical direction (X,Y), and the tilt angle ($\beta$), and/or the position of the hoods (60) in the longitudinal, vertical and transverse direction (X,Y,Z), and the tilt angle ($\alpha$), to the suitable position of the cyclist, and picking up the coordinates and the tilt angle from the movement of the saddle (70) and of the hoods (60), from an initial position to the suitable position of the cyclist, and the coordinates in the longitudinal and vertical direction (X,Y) of the pedals (90);
- a step (S3) for determining the biometric measurements of the contact points (S, M, P) of the cyclist on the bicycle (100) with respect to the crank shaft (11), when the cyclist is in the suitable position based on initial measurements and characteristics of components (MCI) of the bicycle (100), on known measurements of the handlebar assembly adjusting device (200), and/or known measurements of the saddle adjusting device (300), and on the coordinates in the longitudinal direction (X), and in the vertical direction (Y), and the tilt angle ($\beta$) of the movement of the saddle (70), and on the coordinates in the longitudinal direction (X), in the vertical direction (Y), and in the transverse direction (Z), and the tilt angle ($\alpha$) of the movement of the hoods (60), from the initial position to the suitable position of the cyclist; and
- a step for defining final configuration measurements of the bicycle (MBF) and suggested measurements of components (MCF) of the bicycle (S4), which can be measured by the cyclist, based on the biometric measurements and on initial measurements and characteristics of components (MCI) of the bicycle (100), the components the measurements of which are defined in this step (S4) being the stem (30), the handlebar (40), and the seatpost (80), the suggested measurements of the components (MCF) allowing the reproduction of the biometric measurements of the contact points (S, M, P) of the cyclist on the bicycle (100).

14. Method according to claim 13, wherein the input step for entering information (S1) comprises a phase for entering initial configuration measurements of the bicycle (MBI) (P11), equivalent to the final configuration measurements of the bicycle (MBF) when the bicycle (100) is in the initial situation, without installing the system (500), known by the cyclist, these initial configuration measurements of the bicycle (MBI), in step S2, allowing movement of the position of the saddle (70) and of the hoods (60) from the initial position to a starting position defined by the initial configuration measurements of the bicycle (MBI), from where movement of the position of the saddle (70) and of the hoods (60) commences in the movement step (S2), until the suitable position of the cyclist.

15. Method according to claims 13 or 14, wherein the movement step (S2) comprises a phase (P21) for defining the initial position of the saddle (70) and of the hoods (60) after installing the system (500), the coordinates in the longitudinal direction (X) and in the vertical direction (Y), and the tilt angle ($\beta$) of the support point (S) of the saddle (70) being calculated, and the coordinates in the longitudinal direction (X), in the vertical direction (Y), and in the transverse direction (Z), and the tilt angle ($\alpha$) of the support point (H) of the hoods (60) of the initial position, being calculated based on initial measurements and characteristics of components (MCI) of the bicycle (100), on known measurements of the handlebar assembly adjusting device (200), and/or on known measurements of the saddle adjusting device (300), on the hood tilt angle, and on the measurements of the frame (10).

**Patentansprüche**

1. System zur Bestimmung einer geeigneten Position eines Radfahrers auf einem Fahrrad, wobei das Fahrrad (100) der Art ist, umfassend einen Rahmen (10), eine mit dem Rahmen (10) gekoppelte Gabel (20), eine Lenkerbaugruppe (50) umfassend einen Vorbau (30) und einen mit dem Vorbau (30) gekoppelten Lenker (40), wobei der Vorbau (30) mit der Gabel (20) gekoppelt ist, ein Paar Griffe (60), welche mit dem Lenker (40) lösbar gekoppelt sind, einen Sattel (70) und eine Sattelstütze (80), welche an einem Ende mit dem Sattel (70) und am anderen Ende mit dem Rahmen (10) gekoppelt ist, wobei das System (500) eine Lenkerbaugruppeeinstellvorrichtung (200), eine Satteleinstellvorrichtung (300) und Steuer- und Informationsaustauschmittel (400), welche mit der Lenkerbaugruppeeinstellvorrichtung (200) und/oder mit der Satteleinstellvorrichtung (300) verbunden sind, umfasst, **dadurch gekennzeichnet, dass** die Lenkerbaugruppeeinstellvorrichtung (200) dazu ausgebildet ist, die Lenkerbaugruppe (50) des Fahrrads (100) zu ersetzen, wobei die besagte Lenkerbaugruppeeinstellvorrichtung (200) derart ausgebildet ist, dass das Paar Griffe (60) des Fahrrads (100) damit gekoppelt werden können, wobei die besagte Lenkerbaugruppeeinstellvorrichtung (200) dazu ausgebildet ist, mit einem Ende der Gabel gekoppelt zu werden, um eine Kontaktfläche zwischen der Gabel (20) und den Griffen (60) bereitzustellen, und wobei die Lenkerbaugruppeeinstellvorrichtung (200) die Bewegung der Position der Griffe (60) in einer Längsrichtung (X), in einer vertikalen Richtung (Y) und in einer Querrichtung (Z), und mit einem Neigungswinkel ($\alpha$), zur geeigneten Position des Radfahrers auf dem Fahrrad (100) erlaubt.

2. System nach Anspruch 1, wobei die Lenkerbaugruppeeinstellvorrichtung (200) eine Lenkerbaugruppebedieneinheit (210) und eine einstellbare Lenkerbaugruppeneinheit (220), welche mit der Lenkerbaugruppebedieneinheit (210) gekoppelt ist und mit der Gabel (20) gekoppelt ist, umfasst, wobei die Lenkerbaugruppebedieneinheit (210) die einstellbare Lenkerbaugruppeneinheit (220) steuert, um die Bewegung in der vertikalen Richtung (Y) und/oder die Bewegung in der Längsrichtung (X) bereitzustellen, und wobei die einstellbare Lenkerbaugruppeneinheit (220) eine Lenkereinheit (222) umfasst, wobei die Lenkereinheit (222) eine Lenkerhalterung (225), welche mit der Lenkerbaugruppebedieneinheit (210) gekoppelt ist, und ein Lenkerelement (226), welches mit der Lenkerhalterung (225) gekoppelt ist, umfasst, wobei die Griffe (60) des Fahrrads (100) mit dem Lenkerelement (226) gekoppelt sind.

3. System nach Anspruch 2, wobei die Lenkerbaugruppebedieneinheit (210) ein Tiefenstellglied (230) für das Lenkerelement (226) umfasst, um eine Variation der Breite des besagten Lenkerelements (226) in der Querrichtung (Z) bereitzustellen, wobei das Tiefenstellglied (230) mit dem Lenkerelement (226) gekoppelt ist, und wobei das besagte Tiefenstellglied (230) ein manuelles Betätigungselement (231) umfasst, welches in der Lenkerhalterung (225) angeordnet ist.

4. System nach Anspruch 2 oder 3, wobei die Lenkerbaugruppebedieneinheit (210) ein Neigungsstellglied (240) für das Lenkerelement (226) umfasst, um eine Variation des Neigungswinkels ($\alpha$) des besagten Lenkerelements (226) in Bezug auf die Horizontale bereitzustellen, wobei das Neigungsstellglied (240) mit dem Lenkerelement (226) gekoppelt ist, und wobei das besagte Neigungsstellglied (240) ein manuelles Betätigungselement (241), welches in der Lenkerhalterung (225) angeordnet ist, und einen Anzeiger (242), welcher den vom Lenkerelement (226) gedrehten Winkel zeigt und in der Lenkerhalterung (225) angeordnet ist, umfasst.

5. System nach einem der Ansprüche 2 bis 4, wobei die Lenkerbaugruppebedieneinheit (210) ein horizontales Stellglied (211) zur Steuerung der einstellbaren Lenkerbaugruppeneinheit (220), um die Bewegung in der Längsrichtung (X) der Griffe (60) in Bezug auf die Gabel (20) bereitzustellen, und ein vertikales Stellglied (212) zur Steuerung der einstellbaren Lenkerbaugruppeneinheit (220), um die Bewegung in der vertikalen Richtung (Y) der Griffe (60) in Bezug auf die Gabel (20) bereitzustellen, umfasst.

6. System nach Anspruch 5, wobei das horizontale Stellglied (211) und das vertikale Stellglied (212) der Lenkerbaugruppebedieneinheit (210) jeweils mindestens einen Antriebsmotor (213, 214), eine Haltestruktur (215, 216) und ein Linearbewegungselement umfassen, wobei das vertikale Stellglied (212) zusätzlich ein Halteelement (217) umfasst, welches die Haltestruktur (215) des horizontalen Stellglieds (211) hält und mit der Haltestruktur (216) des vertikalen Stellglieds (212) gekoppelt ist, wobei das besagte Halteelement (217) die Kopplung zwischen dem horizontalen Stellglied (211) und dem vertikalen Stellglied (212) der Lenkerbaugruppebedieneinheit (210) erlaubt.

7. System nach Anspruch 5 oder 6, wobei die einstellbare Lenkerbaugruppeneinheit (220) eine Halterung (221) umfasst, wobei die besagte Halterung (221) ein Gabelkopplungselement (223), welches die Kopplung der Halterung (221) mit einem Ende (21) der Gabel (20) des Fahrrads (100) erlaubt, und mindestens einen Arm (224), welcher an einem Ende am Gabelkopplungselement (223) befestigt ist und am anderen Ende das vertikale Stellglied (212) der Lenkerbaugruppebedieneinheit (210) hält, umfasst, wobei die Lenkerhalterung (225) der Lenkereinheit (222) mit dem horizontalen Stellglied (211) der Lenkerbaugruppebedieneinheit (210) gekoppelt ist.

8. System nach einem der vorhergehenden Ansprüche, wobei die Satteleinstellvorrichtung (300) dazu ausgebildet ist, die Sattelstütze (80) des Fahrrads (100) zu ersetzen, wobei die besagte Satteleinstellvorrichtung (300) eine Kontaktfläche zwischen dem Rahmen (10) und dem Sattel (70) bereitstellt, und wobei die Satteleinstellvorrichtung (300) die Bewegung der Position des Sattels (70) in der Längsrichtung (X) und in der vertikalen Richtung (Y), und mit einem Neigungswinkel ($\beta$), zu einer geeigneten Position des Radfahrers auf dem Fahrrad (100) erlaubt, wobei die Satteleinstellvorrichtung (300) eine Sattelstützenbedieneinheit (310) und eine einstellbare Sattelstützeneinheit (320), welche mit der Sattelstützenbedieneinheit (310) gekoppelt und mit dem Rahmen (10) gekoppelt ist, umfasst, wobei der Sattel (70) mit der einstellbaren Sattelstützeneinheit (320) gekoppelt ist, wobei die Sattelstützenbedieneinheit (310) die einstellbare Sattelstützeneinheit (320) steuert, um die Bewegung in der vertikalen Richtung (Y) und/oder die Bewegung in der Längsrichtung (X) bereitzustellen.

9. System nach Anspruch 8, wobei die Sattelstützenbedieneinheit (310) ein Neigungsstellglied (340) für den Sattel (70) umfasst, um eine Variation des Neigungswinkels ($\beta$) des besagten Sattels (70) in Bezug auf die Horizontale bereitzustellen, wobei das Neigungsstellglied (340) einen Antriebsmotor (341) und ein Winkelbewegungselement (342), welches an einem Ende mit dem Antriebsmotor (341) gekoppelt ist und am anderen Ende mit dem Sattel (70) zusammenarbeitet, umfasst.

10. System nach Anspruch 8 oder 9, wobei die Sattelstützenbedieneinheit (310) ein horizontales Stellglied (311) zur Steuerung der einstellbaren Sattelstützeneinheit (320), um die Bewegung in der Längsrichtung (X) des Sattels (70) in Bezug auf den Rahmen (10) bereitzustellen, und ein vertikales Stellglied (312) zur Steuerung der einstellbaren Sattelstützeneinheit (320), um die Bewegung in der vertikalen Richtung (Y) des Sattels (70) in Bezug auf den Rahmen (10) bereitzustellen, umfasst, wobei das horizontale Stellglied (311) und das vertikale Stellglied (312) jeweils mindestens einen Antriebsmotor (313, 314), eine Haltestruktur (315, 316), und eine Linearbewegungselement umfassen, wobei das horizontale Stellglied (311) zusätzlich ein Halteelement (317), welches die Haltestruktur (316) des vertikalen Stellglieds (312) hält und mit der Haltestruktur (315) des horizontalen Stellglieds (311) gekoppelt ist, umfasst, wobei das besagte Halteelement (317) die Kopplung zwischen dem horizontalen Stellglied (311) und dem vertikalen Stellglied (312) der Sattelstützenbedieneinheit (310) erlaubt.

11. System nach Anspruch 10, wobei die einstellbare Sattelstützeneinheit (320) eine Halterung (321) und eine Satteleinheit (322) umfasst, wobei die Halterung (321) ein Rahmenkopplungselement (323), welches die Kopplung der Halterung (321) mit einem Ende (22) des Rahmens (10) des Fahrrads (100) erlaubt, und mindestens einen Arm (324), welcher an einem Ende am Rahmenkopplungselement (323) befestigt ist und am anderen Ende das horizontale Stellglied (311) für die Sattelstützenbedieneinheit (310) hält, umfasst, wobei die Satteleinheit (322) eine Sattelhalterung (325) umfasst, welche mit dem vertikalen Stellglied (312) für die Sattelstützenbedieneinheit (310) gekoppelt ist, und wobei die Sattelhalterung (325) eine Sattelkopplung (326) umfasst, welche an der Sattelhalterung (325) befestigt ist, wobei der Sattel (70) des Fahrrads (100) mit der Sattelkopplung (326) gekoppelt ist.

12. System nach einem der Ansprüche 8 bis 11, wobei die Steuer- und Informationsaustauschmittel (400) eine Steuereinheit (410) zur Steuerung der Lenkerbaugruppeeinstellvorrichtung (200) und der Satteleinstellvorrichtung (300), zur Erfassung der Koordinaten in der Längsrichtung (X), in der vertikalen Richtung (Y) und in der Querrichtung (Z), und des Neigungswinkels ($\alpha$) der Griffe (60), und/oder der Koordinaten in der Längsrichtung (X) und in der vertikalen Richtung (Y), und des Neigungswinkels ($\beta$) des Sattels (70), in Bezug auf eine Kurbelwelle (11) des Fahrrads (100), eine Kontaktfläche (420), welche mit der Steuereinheit (410) verbunden ist, um Auswahlen bezüglich des Systems (500) durchzuführen, um anfängliche Messungen und Merkmale der Bestandteile (MCI) des Fahrrads (100), anfängliche Messungen und Merkmale des Fahrrads (100) selbst, anfängliche Messungen und Merkmale der Fahrradbestandteile und anfängliche Messungen und Merkmale der auf dem Markt bestehende Fahrräder einzugeben (I), und um biometrische Messungen der Kontaktpunkte (S, M, P) des Radfahrers auf dem Sattel (70), auf den Griffen (60) und auf den Pedalen (90) des Fahrrads (100), wenn der Radfahrer in der geeigneten Position ist, endgültige Konfigurationsmessungen des Fahrrads und endgültige Messungen der Bestandteile (MCF) des Fahrrads (100) auszugeben (O), so dass dem Benutzer erlaubt wird, die Koordinaten in der Längsrichtung (X), in der vertikalen Richtung (Y) und in der Querrichtung (Z), sowie den Neigungswinkel ($\alpha$) der Griffe (60) zu ändern, und/oder die Koordinaten in der Längsrichtung (X) und in der vertikalen Richtung (Y), und den Neigungswinkel ($\beta$) des Sattels (70) zu ändern, Datenspeicherungsmittel (430) zur Speicherung der Eingangsinformation (I) und Ausgangsinformation (O), und Verarbeitungsmittel (440) zur Behandlung der Eingangsinformation (I) und zur Bestimmung der Ausgangsinformation (O) umfassen.

13. Verfahren zur Bestimmung einer geeigneten Position eines Radfahrers auf einem Fahrrad, welches mit einem System (500) nach einem der vorhergehenden Ansprüche implementiert ist, **dadurch gekennzeichnet, dass** es Folgendes umfasst:

- einen Eingangsschritt (I) zur Eingabe von Information (S1) über anfängliche Messungen und Merkmale der Bestandteile (MCI) des Fahrrads (100) und über Fahrradbestandteile auf dem Markt, wobei die Bestandteile der Rahmen (10), die Griffe (60), der Sattel (70), die Sattelstütze (80), Pedale (90) und Kurbeln (110) sind, und die eingegebene Information die Messungen der besagten Bestandteile und der Merkmale der besagten Bestandteile, wie die Marke, das Modell, das Herstellungsjahr und die Größe, sind;
- einen Bewegungsschritt (S2) zur Bewegung des Sattels (70) und/oder der Griffe (60) mit dem System (500) auf dem Fahrrad (100) montiert, und mit dem Radfahrer auf dem Fahrrad (100) gestiegen, zur Kopplung der Griffe (60) und des Sattels (70) jeweils mit der Lenkerbaugruppeeinstellvorrichtung (200) und mit der Satteleinstellvorrichtung (300), zur Bewegung der Position des Sattels (70) in der Längs- und vertikalen Richtung (X,Y), und des Neigungswinkels ($\beta$), und/oder der Position der Griffe (60) in der Längs-, vertikalen und Querrichtung (X,Y,Z), und des Neigungswinkels ($\alpha$), zur geeigneten Position des Radfahrers, und zur Erfassung der Koordinaten und des Neigungswinkels aus der Bewegung des Sattels (70) und der Griffe (60), von einer anfänglichen Position zur geeigneten Position des Radfahrers, und der Koordinaten in der Längs- und vertikalen Richtung (X,Y) der Pedale (90);
- einen Schritt (S3) zur Bestimmung der biometrischen Messungen der Kontaktpunkte (S, M, P) des Radfahrers auf dem Fahrrad (100) in Bezug auf die Kurbelwelle (11), wenn der Radfahrer in der geeigneten Position ist, basierend auf anfänglichen Messungen und Merkmalen der Bestandteile (MCI) des Fahrrads (100), auf bekannten Messungen der Lenkerbaugruppeeinstellvorrichtung (200) und/oder bekannten Messungen der Satteleinstellvorrichtung (300), und auf den Koordinaten in der Längsrichtung (X) und in der vertikalen Richtung (Y), und dem Neigungswinkel ($\beta$) der Bewegung des Sattels (70), und auf den Koordinaten in der Längsrichtung (X), in der vertikalen Richtung (Y) und in der Querrichtung (Z), und dem Neigungswinkel ($\alpha$) der Bewegung der Griffe (60), von der anfänglichen Position zur geeigneten Position des Radfahrers; und
- einen Schritt zur Definierung von endgültigen Konfigurationsmessungen des Fahrrads (MBF) und von vorgeschlagenen Messungen der Bestandteile (MCF) des Fahrrads (S4), welche vom Radfahrer gemessen werden können, basierend auf den biometrischen Messungen und auf anfänglichen Messungen und Merkmalen der Bestandteile (MCI) des Fahrrads (100), wobei die Bestandteile von denen die Messungen in diesem Schritt (S4) definiert werden der Vorbau (30), der Lenker (40) und die Sattelstütze (80) sind, wobei die vorgeschlagenen Messungen der Bestandteile (MCF) die Wiedergabe der biometrischen Messungen der Kontaktpunkte (S, M, P) des Radfahrers auf dem Fahrrad (100) erlauben.

14. Verfahren nach Anspruch 13, wobei der Eingangsschritt zur Eingabe der Information (S1) eine Phase zur Eingabe von anfänglichen Konfigurationsmessungen des Fahrrads (MBI) (P11) umfasst, welche äquivalent zu den endgültigen Konfigurationsmessungen des Fahrrads (MBF) sind, wenn das Fahrrad (100) in der anfänglichen Situation ist, ohne das System (500) zu installieren, und für den Radfahrer bekannt sind, wobei diese anfängliche Konfigurationsmessungen des Fahrrads (MBI), in Schritt S2, die Bewegung der Position des Sattels (70) und der Griffe (60) von der anfänglichen Position zu einer Startposition, welche von den anfänglichen Konfigurationsmessungen des Fahrrads (MBI) definiert wird, erlauben, von wo aus die Bewegung der Position des Sattels (70) und der Griffe (60) im Bewegungsschritt (S2) anfängt, bis zur geeigneten Position des Radfahrers.

15. Verfahren nach den Ansprüchen 13 oder 14, wobei der Bewegungsschritt (S2) eine Phase (P21) zur Definierung der anfänglichen Position des Sattels (70) und der Griffe (60) nach der Installation des Systems (500) umfasst, wobei die Koordinaten in der Längsrichtung (X) und in der vertikalen Richtung (Y), und der Neigungswinkel ($\beta$) des Haltepunkts (S) des Sattels (70) berechnet werden, und wobei die Koordinaten in der Längsrichtung (X), in der vertikalen Richtung (Y) und in der Querrichtung (Z), und der Neigungswinkel ($\alpha$) des Haltepunkts (H) der Griffe (60) der anfänglichen Position basierend auf anfänglichen Messungen und Merkmalen der Bestandteile (MCI) des Fahrrads (100), auf bekannten Messungen der Lenkerbaugruppeeinstellvorrichtung (200), und/oder auf bekannten Messungen der Satteleinstellvorrichtung (300), auf dem Neigungswinkel der Griffe, und auf den Messungen des Rahmens (10) berechnet werden.

**Revendications**

1. Système pour déterminer une position appropriée d'un cycliste sur une bicyclette, la bicyclette (100) étant du type comprenant un cadre (10), une fourche (20) couplée au cadre (10), un ensemble de guidon (50) comprenant une potence (30) et un guidon (40) couplé à la potence (30), la potence (30) étant couplée à la fourche (20), une paire de manettes couplées de manière amovible au guidon (40), une selle (70), et une tige de selle (80) couplée à une extrémité de la selle (70), et à l'autre extrémité du cadre (10), le système (500) comprenant un dispositif de réglage de l'ensemble de guidon (200), un dispositif de réglage de selle (300) et des moyens de commande et d'échange d'information (400) connectés au dispositif de réglage de l'ensemble de guidon (200) et/ou au dispositif de réglage de selle (300), **caractérisé en ce que** le dispositif de réglage de l'ensemble de guidon (200) est configuré pour remplacer l'ensemble de guidon (50) de la bicyclette (100), ledit dispositif de réglage de l'ensemble de guidon (200) étant configuré de manière que la paire de manettes (60) de la bicyclette (100) peuvent être couplées à ce dernier, ledit dispositif de réglage de l'ensemble de guidon (200) étant configuré pour être couplé à une extrémité de la fourche pour ainsi fournir une interface entre la fourche (20) et les manettes (60), et le dispositif de réglage de l'ensemble de guidon (20) permettant le déplacement de la position des manettes (60) dans une direction longitudinale (X), dans une direction verticale (Y) et dans une direction transversale (Z), et formant un angle d'inclinaison ($\alpha$), avec la position appropriée du cycliste sur la bicyclette (100).

2. Système selon la revendication 1, dans laquelle le dispositif de réglage de l'ensemble de guidon (200) comprend une unité opérationnelle de l'ensemble de guidon (210), et une unité réglable de l'ensemble de guidon (220) couplée à l'unité opérationnelle de l'ensemble de guidon (210) et couplée à la fourche (20) dans lequel l'unité opérationnelle de l'ensemble de guidon (210) commande l'unité réglable de l'ensemble de guidon (220) pour fournir un déplacement dans la direction vertical (Y) et/ou un déplacement dans la direction longitudinale (X), et dans lequel l'unité réglable de l'ensemble de guidon (220) comprend une unité de guidon (222), l'unité de guidon (222) comprenant un support de guidon (225) couplé à l'unité opérationnelle de l'ensemble de guidon (210), et un élément de guidon (226) couplé au support de guidon (225), les manettes (60) de la bicyclette (100) étant couplées à l'élément de guidon (226).

3. Système selon la revendication 2, dans lequel l'unité opérationnelle de l'ensemble de guidon (210) comprend un actionneur de profondeur (230) pour l'élément de guidon (226) pour fournir une variation dans la largeur dudit élément de guidon (226) dans la direction transversale (Z), l'actionneur de profondeur (230) étant couplé à l'élément de guidon (226), et ledit actionneur de profondeur (230) comprenant un élément d'actionnement manuel (231) aménagé dans le support de guidon (225).

4. Système selon la revendication 2 ou 3, dans lequel l'unité opérationnelle de l'ensemble de guidon (210) comprend un actionneur d'inclinaison (240) pour l'élément de guidon (226), pour fournir une variation dans l'angle d'inclinaison ($\alpha$) dudit élément de guidon (226) par rapport à l'horizontale, l'actionneur d'inclinaison (240) étant couplé à l'élément de guidon (226) et ledit actionneur d'inclinaison (240) comprenant un élément d'actionnement manuel (241) aménagé dans le support de guidon (225), et un indicateur (242) montrant l'angle qui a tourné l'élément de guidon (226), aménagé dans le support de guidon (225).

5. Système selon l'une quelconque des revendications 2 à 4, dans lequel l'unité opérationnelle de l'ensemble de guidon (210) comprend un actionneur horizontal (211) pour commander l'unité réglable de l'ensemble de guidon (220), pour fournir un déplacement dans la direction longitudinale (X) des manettes (60) par rapport à la fourche (20), et un actionneur vertical (212) pour commander l'unité réglable de l'ensemble de guidon (220), pour fournir un déplacement dans la direction verticale (Y) des manettes (60) par rapport à la fourche (20).

6. Système selon la revendication 5, dans lequel l'actionneur horizontal (211) et l'actionneur vertical (212) de l'unité opérationnelle de l'ensemble de guidon (210) comprennent chacun au moins un moteur d'entraînement (213, 214), une structure de support (215, 216), et un élément de déplacement linéaire, respectivement, l'actionneur vertical (212) comprenant en outre un élément de support (217), supportant la structure de support (215) de l'actionneur horizontal (211) couplé à la structure de support (216) de l'actionneur vertical (212) ledit élément de support (217) permettant l'accouplement entre l'actionneur horizontal (211) et l'actionneur vertical (212) de l'unité opérationnelle de l'ensemble de guidon (210).

**7.** Système selon la revendication 5 ou 6, dans lequel l'unité réglable de l'ensemble de guidon (220) comprend un support (221), ledit support (221) comprenant un élément d'accouplement de fourche (223) qui permet l'accouplement du support (221) à une extrémité (21) de la fourche (20) de la bicyclette (100), et au moins un bras (224) fixé par une extrémité à l'élément d'accouplement de fourche (223), et supportant à l'autre extrémité l'actionneur vertical (212) de l'unité opérationnelle de l'ensemble de guidon (210), le support de guidon (225) de l'unité de guidon (222) étant couplé à l'actionneur horizontal (211) de l'unité opérationnelle de l'ensemble de guidon (210).

**8.** Système selon l'une quelconque des revendications précédentes, dans lequel le dispositif de réglage de selle (300) est configuré pour remplacer la tige de selle (80) de la bicyclette (100), ledit dispositif de réglage de selle (300) fournissant une interface entre le cadre (10) et la selle (70), et le dispositif de réglage de selle (300) permettant un déplacement de la position de la selle (70) dans la direction longitudinale (X) et dans la direction verticale (Y), et formant un angle d'inclinaison ($\beta$), vers une position appropriée du cycliste sur la bicyclette (100), le dispositif de réglage de la selle (300) comprenant une unité opérationnelle de tige de selle (310), et une unité de réglage de tige de selle (320) couplée à l'unité opérationnelle de tige de selle (310) et couplé au cadre (10), la selle (70) étant couplée à l'unité réglable de tige de selle (320), dans lequel l'unité opérationnel de tige de selle (310) commande l'unité réglable de tige de selle (320) pour fournir un déplacement dans la direction verticale (Y) et/ou un déplacement dans la direction longitudinale (X).

**9.** Système selon la revendication 8, dans lequel l'unité opérationnelle de tige de selle (310) comprend un actionneur d'inclinaison (340) pour la selle (70), pour fournir une variation dans l'angle d'inclinaison ($\beta$) de ladite selle (70) par rapport à l'horizontale, l'actionneur d'inclinaison (340) comprenant un moteur d'entraînement (341) et un élément de déplacement angulaire (342) couplé par une extrémité au moteur d'entraînement (341) et coopérant à l'autre extrémité avec la selle (70).

**10.** Système selon la revendication 8 ou 9, dans lequel l'unité opérationnelle de tige de selle (310) comprend un actionneur horizontal (311) pour commander l'unité réglable de tige de selle (320) pour fournir un déplacement dans la direction longitudinale (X) de la selle (70) par rapport au cadre (10), et un actionneur vertical (312) pour commander l'unité réglable de tige de selle (320) pour fournir un déplacement dans la direction verticale (Y) de la selle (70) par rapport au cadre (10), l'actionneur horizontal (311) et l'actionneur vertical (312) comprenant chacun au moins un moteur d'entraînement (313, 314), une structure de support (315, 316), et un élément de déplacement linéaire, respectivement, l'actionneur horizontal (311) comprenant en outre un élément de support (317) supportant la structure de support (316) de l'actionneur vertical (312), couplé à la structure de support (315) de l'actionneur horizontal (311), ledit élément de support (317) permettant l'accouplement entre l'actionneur horizontal (311) et l'actionneur vertical (312) de l'unité opérationnelle de tige de selle (310).

**11.** Système selon la revendication 10, dans lequel l'unité réglable de tige de selle (320) comprend un support (321) et une unité de selle (322), le support (321) comprenant un élément d'accouplement de cadre (323) qui permet l'accouplement du support (321) à une extrémité (22) du cadre (10) de la bicyclette (100), et au moins un bras (324) fixé par une extrémité à l'élément d'accouplement de cadre (323), et supporte à une autre extrémité l'actionneur horizontal (311) pour l'unité opérationnelle de tige de selle (310), l'unité de selle (322) comprenant un support de selle (325) couplé à l'actionneur vertical (312) pour l'unité opérationnelle de tige de selle (310), et le support de selle (325) comprenant un accouplement de selle (326) fixé au support de selle (325), la selle (70) de la bicyclette (100) étant couplée à l'accouplement de selle (326).

**12.** Système selon l'une quelconque des revendications 8 à 11, dans lequel les moyens de commande et d'échange d'information (400) comprennent une unité de commande (410) pour commander le dispositif de réglage de l'ensemble de guidon (200) et le dispositif de réglage de selle (300), pour recueillir les coordonnées dans la direction longitudinale (X), dans la direction verticale (Y), et dans la direction transversale (Z), et l'angle d'inclinaison ($\alpha$) des manettes (60), et/ou les coordonnées dans la direction longitudinale (X) et dans la direction verticale (Y), et l'angle d'inclinaison ($\beta$) de la selle (70) par rapport à une bielle (11) de la bicyclette (100), une interface (420) connectée à l'unité de commande (410) pour réaliser des sélections concernant le système (500), pour introduire (I) des mesures et des caractéristiques initiales des composants (MCI) de la bicyclette (100), des mesures et des caractéristiques initiales de la bicyclette (100) elle-même, des mesures et des caractéristiques initiales des composants de bicyclette et des mesures et des caractéristiques initiales de bicyclettes existant sur le marché, et pour produire (O) des mesures biométriques des points de contact (S, M, P) du cycliste sur la selle (70), sur les manettes (60), et sur les pédales (90) de la bicyclette (100), lorsque le cycliste est dans la position appropriée, des mesures de configuration finales du cycliste, et des mesures finales des composants (MCF) de la bicyclette (100), permettant que l'utilisateur modifie les coordonnées dans la direction longitudinale (X), dans la direction verticale (Y), et dans la direction transversale (Z), ainsi que l'angle d'inclinaison (α) des manettes (60), et/ou qu'il modifie les coordonnées dans la direction longitudinale (X) et dans la direction verticale (Y), et l'angle d'inclinaison (β) de la selle (70), des moyens de stockage de données (430) pour stocker l'information d'entrée (I) et l'information de sortie (O), et des moyens de traitement (440) pour traiter l'information d'entrée (I) et déterminer l'information de sortie (O).

**13.** Procédé pour déterminer une position appropriée d'un cycliste sur une bicyclette, mis en oeuvre par un système (500) selon l'une quelconque des revendications précédentes, **caractérisé en ce qu'**il comprend :

- une étape d'entrée (I) pour introduire information (S1) sur des mesures et caractéristiques initiales des composants (MCI) de la bicyclette (100) et sur des composants de bicyclette sur le marché, les composants étant le cadre (10), les manettes (60), la selle (70), la tige de selle (80), les pédales (90), et les bielles (110), et l'information introduite est les mesures desdits composants et les caractéristiques desdits composants, comme le fabricant, le modèle, l'année de fabrication et les dimensions ;
- une étape de déplacement (S2) pour déplacer la selle (7') et/ou les manettes (60) avec le système (500) monté sur la bicyclette (100), et avec le cycliste monté sur la bicyclette (100), accouplant les manettes (60) et la selle (70) au dispositif de réglage de l'ensemble de guidon (200), et au dispositif de réglage de selle (300), respectivement, déplacer la position de la selle (70) dans la direction longitudinale et verticale (X, Y), et l'angle d'inclinaison (β), et/ou la position de manettes (60) dans la direction longitudinale, verticale et transversale (X, Y, Z), et l'angle d'inclinaison (α), vers la position appropriée du cycliste, et recueillir les coordonnées et l'angle d'inclinaison du déplacement de la selle (70) et des manettes (60), d'une position initiale à la position appropriée du cycliste, et les coordonnées dans la direction longitudinale et verticale (X, Y) des pédales (90) ;
- une étape (S3) pour déterminer des mesures biométriques des points de contact (S, M, P) du cycliste sur la bicyclette (100) par rapport à la bielle (11), lorsque le cycliste est dans la position appropriée basée sur les mesures et caractéristiques initiales des composants (MCI) de la bicyclette (100), sur des mesures connues du dispositif de réglage de l'ensemble de guidon (200), et/ou des mesures connues du dispositif de réglage de selle (300), et sur les coordonnées dans la direction longitudinale (X), et dans la direction verticale (Y), et l'angle d'inclinaison (β) du déplacement de la selle (70), et sur les coordonnées dans la direction longitudinale (X), dans la direction verticale (Y), et dans la direction transversale (Z), et l'angle d'inclinaison (α) du déplacement des manettes (60), de la position initiale à la position appropriée du cycliste ; et
- une étape de définition des mesures de configuration finales de la bicyclette (MBF) et des mesures suggérées des composants (MCF) de la bicyclette (S4), qui peuvent être mesurées par le cycliste, basées sur les mesures biométriques et sur les mesures et caractéristiques initiales des composants (MCI) de la bicyclette (100), les composants dont les mesures sont définies dans l'étape (S4) étant la potence (30), le guidon (40), et la tige de selle (80), les mesures suggérées des composants (MCF) permettant la reproduction des mesures biométriques des points de contact (S, M, P) du cycliste sur la bicyclette (100).

**14.** Procédé selon la revendication 13, dans lequel l'étape d'entrée pour introduire information (S1) comprend une phase pour introduire des mesures de configuration initiales de la bicyclette (MBI) (P11), équivalant aux mesures de configuration finales de la bicyclette (MBF) lorsque la bicyclette (100) est dans la situation initiale, sans installer le système (500), connues par le cycliste, ces mesures de configuration initiales de la bicyclette (MBI), dans l'étape S2, permettant le déplacement de la position de la selle (70) et des manettes (60) de la position initiale à une position de départ définie par les mesures de configuration initiales de la bicyclette (MBI), où commence le déplacement de la position de la selle (70) et des manettes (60) dans l'étape de déplacement (S2), jusqu'à la position approprié du cycliste.

**15.** Procédé selon les revendications 13 ou 14, dans lequel l'étape de déplacement (S2) comprend une phase (P21) pour définir la position initiale de la selle (70) et des manettes (60) après l'installation du système (500), les coordonnées dans la direction longitudinale (X) et dans la direction verticale (Y), et l'angle d'inclinaison (β) du point de support (S) de la selle (70) étant calculées, et les coordonnées dans la direction longitudinale (X), dans la direction verticale (Y), et dans la direction transversale (Z), et l'angle d'inclinaison (α) du point de support (H) des manettes de la position initiale, étant calculées sur la base des mesures et caractéristiques initiales des composants (MCI) de la bicyclette (100), sur des mesures connues du dispositif de réglage de l'ensemble de guidon (200), et/ou sur des mesures connues du dispositif de réglage de selle (300), sur l'angle d'inclinaison des manettes, et sur les mesures du cadre (10).

FIG. 1

**FIG. 2**

**FIG. 3**

FIG. 4

EP 2 979 625 B1

FIG. 5a

FIG. 5b

FIG. 5c

FIG. 6a

FIG. 6b

FIG. 6c

FIG. 7

FIG. 8

FIG. 9

FIG. 10

FIG. 11

FIG. 12

**FIG. 13**

**FIG. 14**

EP 2 979 625 B1

FIG. 15

EP 2 979 625 B1

FIG. 16

FIG. 17

EP 2 979 625 B1

FIG. 18

FIG. 19

FIG. 20

FIG. 21

# REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

## Patent documents cited in the description

- US 20120202653 A

- WO 2014066994 A1